Europäisches Patentamt

European Patent Office

Office européen des brevets

(11) Publication number: **0 258 221**
**B1**

## (12) EUROPEAN PATENT SPECIFICATION

(45) Date of publication of patent specification: **16.05.90**

(21) Application number: **86901236.9**

(22) Date of filing: **29.01.86**

(86) International application number:
**PCT/US86/00211**

(87) International publication number:
**WO 87/04627 13.08.87 Gazette 87/18**

(51) Int. Cl.⁵: **A 61 M 1/00, A 61 M 25/00**

## (54) COAXIAL ARTERIOVENOUS GRAFTING TUBE.

(43) Date of publication of application:
**09.03.88 Bulletin 88/10**

(45) Publication of the grant of the patent:
**16.05.90 Bulletin 90/20**

(84) Designated Contracting States:
**AT BE CH DE FR GB IT LI LU NL SE**

(56) References cited:
**FR-A-2 215 248
FR-A-2 366 843
US-A-3 112 748**

(73) Proprietor: **MOUNT SINAI SCHOOL OF
MEDICINE OF THE CITY UNIVERSITY OF NEW
YORK
One Gustave L. Levy Place
New York, NY 10029 (US)**

(72) Inventor: **SCHANZER, Harry
9 Larchmont Avenue
Larchmont, NY 10538 (US)**

(74) Representative: **Lucas, Brian Ronald et al
Lucas, George & Co. 135 Westhall Road
Warlingham Surrey CR3 9HJ (GB)**

Courier Press, Leamington Spa, England.

**Description**

This invention relates to a tube for use as a vascular graft to permit in vivo hemoaccess.

Background of the Invention

Dialysis treatment for patients with renal failure require a ready access to blood vessels in order for blood to be continuously withdrawn from the patient in amounts of over 200 ml/min. The blood withdrawn from the patient is passed through a dailysis machine, and returned to the patient. A very common method of producing this hemoaccess is the use of expanded polytetrafluorethylene (PTFE) tubes as grafts which are surgically placed between an artery and a vein (PTFE AV fistula). This procedure is especially useful in patients who do not have blood vessels which will support the construction of a more traditional primary fistula on the forearm.

These PTFE grafts cannot be used safely to withdraw blood however until they have been in place for a minimum of 14 days after surgery and have become surrounded by fibrotic tissue. Because of the bleeding which occurs at the site of a needle puncture in these grafts if fibrotic tissue is absent, common complications encountered with early puncturing of PTFE AV fistulas are a hematoma surrounding the graft, false aneurysm and possible graft occlusion. The Atlas of Angioaccess Surgery by Pedro A. Rubio and Edward M. Farrell (Year Book Medical Publishers, Inc., 1983) states that "graft fistulas should not be punctured before the third postoperative week. Earlier puncture risks hematoma formation in the subcutaneous tunnel following needle withdrawal, which will result in further delay in healing." Various materials in addition to PTFE have been tried including autologous saphenous vein, Dacron synthetic polyester fiber velour, modified bovine carotid xenograft, and modified human umbilical vein, but none have overcome the problems associated with early puncture of the graft.

A coaxial double tube for use in hemoaccess wherein the space between the two tubes is filled with a self-sealing, nonbiodegradable polymer (hereinafter "filled double tubes") has now been discovered. The filled double tube does not bleed upon puncture with a dialysis needle even immediately after surgery. Ex vivo perfusion tests and AV fistula experiments in dogs clearly show that the filled double tubes are superior as grafts to the known single PTFE tube in the amount of bleeding resulting from puncture of the tube with a dialysis needle.

The advantages of the filled double tube are that (1) it can be used for hemoaccess immediately after implantation, (2) the lack of bleeding results in a lower incidence of perigraft hematomas and a lower incidence of pseudoaneurysms, (3) the lack of bleeding upon puncture obviates the need for prolonged compression of the puncture site with secondary occlusion of the tube, and (4) due to the increased rigidity of the filled double tube, puncturing of the tube is easier.

FR-A-2 215 248 discloses a double tube having improved leakage resistance which is intended for use as part of the conduits of a dialysis or similar device but which is not for implantation.

Summary of the Invention

The present invention provides a tube for use as a vascular graft to permit in vivo hemoaccess, which tube comprises an outer tube positioned over an inner tube, wherein the outer tube has an internal diameter which is at least 1 mm larger than the external diameter of the inner tube creating a space between the outer tube and the inner tube and that a self-sealing material evenly fills the space between the outer tube and the inner tube, said material comprising a nonbiodegradable, biocompatible polymer adhesive.

Brief Description of the Drawing

Figure 1 is a plan view of the claimed filled double tube;
Figure 2 is a cross-sectional view of the claimed filled double tube; and
Figure 3 is a chart which compares the blood loss from the filled double tube when used as a graft for hemoaccess with the blood loss from other conventional grafts.

Description of Preferred Embodiment

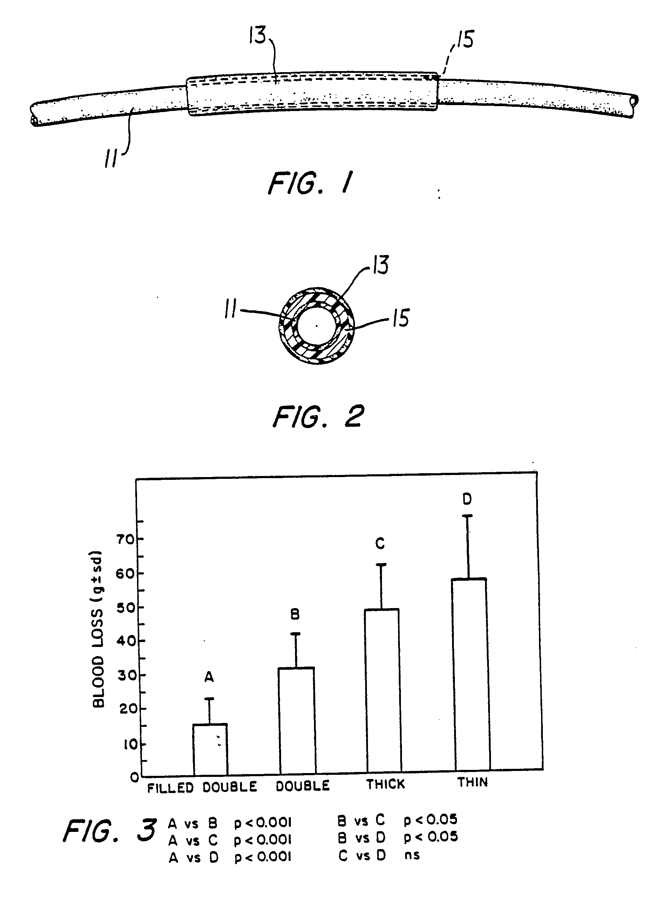

As illustrated in Figures 1 and 2, the filled double tube comprises an inner tube 11 and an outer tube 13 which is positioned over the center portion of the inner tube leaving a space 15 between the inner and outer tube 13 and allowing the ends of the inner tube to extend equally beyond the ends of the outer tube. The space 15 is filled with a self-sealing, nonbiodegradeable, biocompatible polymer (hereinafter "polymer") for example a silicone rubber sealant (Dow Corning Catalogue No. 732-3).

The inner tube 11 can have an internal diameter from about 4 mm to 8 mm with 6 mm being the preferred internal diameter and a wall thickness of about 0.5 to 1 mm. The outer tube 13 can have an internal diameter from about 6 mm to 10 mm which is at least 1 mm larger than the external diameter (internal diameter + wall thickness) of the inner tube with 8 mm being the preferred internal diameter and a wall thickness of about 0.5 mm to 1 mm, preferably 1 mm. The wall thickness of the inner and outer tubes may be the same or different.

The length of the inner tube 11 depends on the specific application for the tube, and the site in the patient at which the filled double tube is implanted. As a result, the inner tube 11 can vary in length from 10 mm to 500 mm with 150 mm being the preferred length for looped forearm grafts. The length of the outer tube 13 is equal to or less than that of the inner tube 11 and can vary in length from 60 mm to 400 mm with 100 mm being the preferred length for forearm grafts. The inner tube 11 can be made from any hemocompatible material suitable as a conduit. Suitable material for the inner and outer tubes are, for example, Teflon PTFE and Dacron synthetic polyester fiber. The outer tube 13 can be made from any biocompatible material suitable as a conduit. Examples of suitable materials are Teflon PTFE and Dacron synthetic polyester fiber. Preferably, the filled double tube is made entirely from GORE-TEX reinforced PTFE tubing developed by W. F. Gore & Associates, Inc. by rapidly stretching highly crystalline unsintered polytetrafluorethylene.

The filled double tube can be used for any medical technique in which repeated hemoaccess is required, for example, but without intending to limit the possible applications, intravenous drug administration, chronic insulin injections, chemotherapy, frequent blood samples, connection to artificial lungs and hyperalimentation.

The filled double tube is ideally suited for use in chronic hemodialysis access for example in a looped forearm graft fistula, straight forearm graft fistula, straight arm graft fistula, looped thigh graft fistula and axillaryaxillary graft fistula or any other AV fistula application. Because of its hard consistency and resistance to collapse, the filled double tube may also be useful for among other things, arterial bypasses subjected to external pressure (for example, axillo-femoral, axillo-axillo bypasses) and other replacement surgery.

In an alternate embodiment of the invention, the inner surface of the outer tube and the outer surface of the inner tube may be rough to prevent possible separation of the polymer from the tube surface during use.

The filled double tubes used in the following Examples (2-3) were prepared by the procedures outlined in Example 1. The same procedure can be used to prepare filled double tubes of various sizes and lengths.

Example 1

A 150 mm segment of a 6 mm internal diameter thick GORE-TEX reinforced PTFE tube was placed over a metallic rod. The rod had a diameter of 6 mm and fit the lumen of the tube tightly. A 70 mm segment of a 8 mm internal diameter thick GORE-TEX reinforced PTFE tube was placed over the inner tube with the rod in its lumen. The outer tube was centered over the inner tube in order to leave about 40 mm of the inner tube free on both ends.

One of the ends of the outer tube was sealed with silicone rubber sealant (Dow Corning Cat. #732-3) by using a 5 ml syringe filled with silicone sealant and equipped with an angiocatheter. After allowing the tubes to stand for 24 hours the sealant was cured and the opening at one end was completely sealed. The remaining space between the inner and outer tubes was filled by diluting silicone sealant 3:1 (w/v) with acetic acid and placing the diluted silicone sealant in a 5 cc syringe equipped with a 2 mm diameter catheter, The catheter was inserted into the remaining space between the tubes from the unsealed end and the syringe was placed in a Harvard pump.

The pump injected the silicone into the unsealed end and the catheter was slowly withdrawn. As soon as excess sealant began to appear at the open end, injection was discontinued, The filled double tube was then rolled on a smooth surface to distribute the silicone uniformly in the space between the tubes and after setting for an additional 24 hours the silicone sealant was cured and the silicone filled double tube was removed from the metallic rod and was ready for use.

Example 2

A 60 mm double tube having as an inner tube a 4 mm internal diameter GORE-TEX PTFE thin tube and as an outer tube a coaxial 6 mm thick GORE-TEX PTFE tube with the space between the tubes filled with silicone rubber sealant ("filled double") was employed. For comparison the following control tubes of the same lengths were used: (1) an unfilled double tube using a 4 mm inner thin wall GORE-TEX PTFE tube and a 6 mm outer thick wall GORE-TEX PTFE tube without silicone in the space between the tubes ("double"), (2) a thin 4 mm conventional GORE-TEX PTFE tube ("thin"), and (3) a thick 6 mm conventional GORE-TEX PTFE tube ("thick"). Four dogs were used in this experiment.

A Scribner shunt was placed between the femoral artery and the femoral vein in each dog. Each of the tubes to be tested was placed between the arterial and venous branches of the shunts. The four different types of tubes were inserted three times in various sequences in each dog (12 measurements per dog).

Puncturing was performed in each case by piercing the tube with an 18-gauge needle. Intraluminal pressure measurements were carried out with each puncture in order to be certain that the tube was open and that the pressure condition remained constant. After removing the needle, each tube puncture site was allowed to bleed freely into a cup for 30 seconds. The Scribner shunt was then clamped and the tube changed. The amount of bleeding was measured by weighing the cup before and after bleeding. The results of these experiments are set forth in Tables 1, 2 and 3 and Figure 3 and clearly demonstrate a very significant reduction in bleeding after puncture ex vivo using the new filled double tube ("filled double") for the graft.

## TABLE 1

### HEMODIALYSIS ACCESS (GORE-TEX® PTFE GRAFTS)

#### Ex-Vivo Bleeding Tests

|          | Graft Type    | Blood Pressures          | Blood Weights      | m ± sd      |
|----------|---------------|--------------------------|--------------------|-------------|
| Leg # 1  | Thin          | 125/80, 125/95, 115/85   | 53.5, 100, 63.5    | 72.3 ± 24.5 |
| Dog # 1  | Thick         | 210/120, 125/80, 140/110 | 46.0, 34.9, 64.9   | 48.6 ± 15.2 |
|          | Double        | 115/60, 115/75, 113/95   | 42.6, 47.4, 35.4   | 41.8 ± 6.0  |
|          | Filled Double | 110/80, 100/75, 115/85   | 10.9, 23.5, 6.3    | 13.6 ± 8.9  |
| Leg # 2  | Thin          | 130/90, 164/120, 160/110 | 47.7, 44.6, 51.7   | 48.0 ± 3.5  |
| Dog # 2  | Thick         | 120/80, 140/100, 150/100 | 57.1, 57.0, 60.0   | 58.0 ± 1.7  |
|          | Double        | 140/100, 140/100, 160/140| 36.3, 27.2, 24.7   | 29.4 ± 6.1  |
|          | Filled Double | 135/80, 140/100, 160/120 | 11.7, 24.1, 1.7    | 12.5 ± 11.2 |
| Leg # 3  | Thin          | 95/70, 115/70, 120/70    | 54.7, 37.2, 61.3   | 51.0 ± 12.4 |
| Dog # 3  | Thick         | 90/70, 115/70, 95/60     | 37.7, 41.4, 41.5   | 40.2 ± 2.2  |
|          | Double        | 85/55, 110/70, 115/70    | 29.5, 38.0, 53.4   | 40.3 ± 12.1 |
|          | Filled Double | 80/50, 80/60, 115/70     | 16.7, 13.5, 18.9   | 16.4 ± 2.7  |
| Leg # 4  | Thin          | 130/85, 120/75, 100/60   | 43.3, 34.4, 28.4   | 35.4 ± 7.5  |
| Dog # 4  | Thick         | 135/80, 115/70, 90/65    | 54.1, 32.2, 28.6   | 38.3 ± 13.8 |
|          | Double        | 135/80, 125/75, 120/75   | 37.1, 19.7, 30.5   | 27.1 ± 8.8  |
|          | Filled Double | 125/90, 110/80, 95/65    | 22.0, 13.3, 26.3   | 20.5 ± 6.6  |

EP 0 258 221 B1

## TABLE 2

### EX-VIVO PERFUSION BLEEDING TESTS

| Dog # | Graft Type | Blood Pressure | Blood Weight | m = sd |
|-------|------------|----------------|--------------|--------|
| 1 | Thin | 125/80, 125/95, 115/85 | 53.5, 100, 63.5 | |
| 2 | | 130/90, 164/120, 160/110 | 47.7, 44.6, 51.7 | |
| 3 | .. | 95/70, 115/70, 120/70 | 54.7, 37.2, 61.3 | 51.7 = 18.5 |
| 4 | | 130/85, 120/75, 100/60 | 43.3, 34.4, 28.4 | |
| 1 | Thick | 210/120, 125/80, 140/110 | 46.0, 34.9, 64.9 | |
| 2 | | 120/80, 140/100, 150/100 | 57.1, 57.0, 60.0 | |
| 3 | | 90/70, 115/70, 95/60 | 37.7, 41.4, 41.5 | 43.2 = 16.0 |
| 4 | | 135/80, 115/70, 90/65 | 54.1, 32.5, 28.6 | |
| 1 | Double | 115/60, 115/75, 113/95 | 42.6, 47.4, 35.4 | |
| 2 | | 140/100, 140/100, 160/140 | 36.3, 27.2, 24.7 | |
| 3 | | 85/55, 110/70, 115/70 | 29.5, 38.0, 53.4 | 31.5 = 9.6 |
| 4 | | 135/80, 125/75, 120/75 | 37.1, 19.7, 30.5 | |
| 1 | Filled Double | 110/80, 100/75, 115/85 | 10.9, 23.5, 6.3 | |

EP 0 258 221 B1

## TABLE 3

| GRAFT | | THIN | THICK | DOUBLE | FILLED DOUBLE |
|---|---|---|---|---|---|
| | | (grams ± s.d.)** | | | |
| | | 51.7 ± 18.5* | 43.2 ± 16.0 | 31.5 ± 9.6 | 15.6 ± 7.5 |
| STATISTICS | | | | | |
| | | | N | t | P |
| Filled Double | vs. Double | | 22 | 5.50 | <0.001 |
| Filled Double | vs. Thin | | 22 | 6.11 | <0.001 |
| Filled Double | vs. Thick | | 22 | 7.48 | <0.001 |
| Thin | vs. Double | | 22 | 2.53 | <0.05 |
| Thick | vs. Double | | 22 | 2.52 | <0.05 |
| Thin | vs. Thick | | 22 | 0.74 | n.s. |

*mean of 12 measurements in 4 legs (3 measurements/leg).

**grams of blood in 30 seconds from an 18-gauge needle puncture of the tubes.

Example 3

Eleven dogs had a fistula constructed between the right carotid artery and the left external jugular vein, using a graft going through a subcutaneous tunnel in the posterior aspect of the neck. A 6 mm diameter conventional GORE-TEX PTFE tube 120 mm in length was used in 5 dogs and a 120 mm filled double tube prepared according to Example 1 was tested on 6 dogs. Each graft was punctured on days 1, 3-4, and 7-8 after construction of the fistula. Five minutes after removing the needle, a small incision over the puncture area was made, thereby exposing the tube. The amount of blood present was estimated by absorbing it with sponges and weighing them. The results are shown in Table 4.

In the control group, 8 punctures resulted in the loss of over 100 ml of blood. Two punctures bled between 40 and 100 ml, 2 punctures bled 20 ml and 40 ml and only one puncture bled less than 20 ml. In 10 instances, it was necessary to place a stitch in the tube in order to stop the bleeding, In the filled double tube, only 3 out of 14 punctures bled. Two bled between 20 ml and 40 ml and the third one bled less than 20 ml, Not one puncture required stitches for hemostasis.

The results described in Examples 2 and 3 clearly demonstrate the superior characteristics of the filled double tube over conventional grafts for hemoaccess in terms of the amount of bleeding resulting from a needle puncture. This was clearly verified both in the ex vivo experiment (Example 2) and in the in vivo interposition AV fistulas (Example 3).

## Table 4

| Dog # | Graft Type | Days of Puncture | | |
|-------|------------|------|-----|-----|
| | | 1 | 3-4 | 7-8 |
| 8006 | 6 mm Gore-Tex® | ++++* | ++++* | clotted |
| 8060 | " | ++++* | ++* | + |
| 8064 | " | ++++ | ++++* | ++++* |
| 8065 | " | ++++* | ++++* | clotted |
| 8103 | " | ++ | +++* | +++* |
| 8005 | Double "silicone" | 0 | 0 | 0 |
| 8056 | " | ++ | 0 | 0 |
| 8061 | " | 0 | 0 | died |
| 8101 | " | 0 | + | clotted |
| 8102 | " | 0 | clotted | |
| 8099 | " | 0 | 0 | ++ |

```
0:   no bleeding
+:   0-20 ml of blood
++:  2-40 ml of blood
+++: more than 100 ml of blood
*:   needle hole required stitch to control bleeding
```

## Claims

1. A tube for use as a vascular graft to permit in vivo hemoaccess, which tube comprises an outer tube (13) positioned over an inner tube (11), wherein the outer tube has an internal diameter which is at least 1 mm larger than the external diameter of the inner tube (11) creating a space between the outer tube and the inner tube and that a self-sealing material evenly fills the space between the outer tube and the inner tube, said material comprising a nonbiodegradable, biocompatible polymer adhesive.

2. A tube as claimed in Claim 1, characterized in that the outer tube (13) is shorter than the inner tube (11) and is positioned over the centre of the inner tube (11) thereby allowing the ends of the inner tube (11) to extend beyond each end of the outer tube (13).

3. A tube as claimed in Claim 1 or 2, characterized in that the inner tube (11) and outer tubes (13) are made from a material selected from the group consisting of PTFE, synthetic polyester fibre, and reinforced PTFE and the self-sealing, nonbiodegradable, biocompatible polymer is a silicone sealant.

4. A tube as claimed in Claim 3, characterized in that the outer tube (13) is synthetic polyester fibre and the inner tube (11) is reinforced PTFE.

5. A tube as claimed in any preceding Claim characterized in that the outer tube has an internal diameter of about 6 mm to about 10 mm and a wall thickness of about 0.5 mm to 1 mm, and the inner tube has an internal diameter of about 4 mm to about 8 mm and a wall thickness of about 0.5 mm to about 1 mm.

6. A tube as claimed in Claim 5, characterized in that the outer tube has an internal diameter of about 8 mm and a wall thickness of about 1 mm and the inner tube has an internal diameter of about 6 mm and a wall thickness of about 1 mm.

7. A tube as claimed in any preceding Claim, characterized in that the polymer adhesive has been introduced into the space between the outer and inner tubes and has cured in situ.

**Patentansprüche**

1. Schlauch zur Verwendung als Gefäßtransplantat, um den Zutritt zur Blutbahn in vivo zu ermöglichen, wobei dieser Schlauch einen Außenschlauch (13) umfaßt, der über einem Innenschlauch (11) angeordnet ist, wobei der Außenschlauch einen Innendurchmesser aufweist, der mindestens 1 mm größer als der Außendurchmesser des Innenschlauchs (11) ist, wobei zwischen dem Außenschlauch und dem Innenschlauch ein Raum gebildet wird und ein selbstabdichtendes Material den Raum zwischen dem Außenschlauch und dem Innenschlauch gleichmäßig füllt, wobei dieses Material ein Klebemittel in Form eines biologisch nicht abbaubaren, bioverträglichen Polymers umfaßt.

2. Schlauch nach Anspruch 1, dadurch gekennzeichnet, daß der Außenschlauch (13) kürzer als der Innenschlauch (11) ist und über der Mitte des Innenschlauchs (11) angeordnet ist, wodurch sich die Enden des Innenschlauchs (11) über jedes Ende des Außenschlauchs (13) erstrecken.

3. Schlauch nach Anspruch 1 oder 2, dadurch gekennzeichnet, daß der Innenschlauch (11) und der Außenschlauch (13) aus einem Material gefertigt sind, das aus der Gruppe ausgewählt ist, die aus PTFE, synthetischer Polyesterfaser und verstärktem PTFE besteht, und das selbstabdichtende, biologisch nicht abbaubare, bioverträgliche Polymer ein Silikondichtmittel ist.

4. Schlauch nach Anspruch 3, dadurch gekennzeichnet, daß der Außenschlauch (13) eine synthetische Polyesterfaser und der Innenschlauch (11) verstärktes PTFE ist.

5. Schlauch nach einem der vorstehenden Ansprüche, dadurch gekennzeichnet, daß der Außenschlauch einen Innendurchmesser von etwa 6 mm bis etwa 10 mm und eine Wanddicke von etwa 0,5 mm bis 1 mm aufweist und der Innenschlauch einen Innendurchmesser von etwa 4 mm bis etwa 8 mm und eine Wanddicke von etwa 0,5 mm bis etwa 1 mm aufweist.

6. Schlauch nach Anspruch 5, dadurch gekennzeichnet, daß der Außenschlauch einen Innendurchmesser von etwa 8 mm und eine Wanddicke von etwa 1 mm und der Innenschlauch einen Innendurchmesser von etwa 6 mm und eine Wanddicke von etwa 1 mm hat.

7. Schlauch nach einem der vorstehenden Ansprüche, dadurch gekennzeichnet, daß das Polymerklebemittel in den Raum zwischen dem Außen- und dem Innenschlauch angebracht wurde und in situ gehärtet wurde.

**Revendications**

1. Tube pour utilisation comme greffe vasculaire pour permettre l'hémoaccès in vivo, lequel tube comprend un tube externe (13) positionné sur un tube interne (12) où le tube externe a un diamètre interne qui est d'au moins 1 mm supérieur au diamètre externe du tube interne (11) créant un espace entre le tube externe et le tube interne, et où une matière auto-scellante remplit de manière régulière l'espace compris entre le tube externe et le tube interne, ladite matière comprenant un additif polymérique biocompatible non biodégradable.

2. Tube selon la revendication 1, caractérisé en ce que le tube externe (13) est plus court que le tube interne (11) et est positionné au-dessus du centre du tube interne (11), permettant ainsi aux extrémités du tube interne (11) de s'étendre au-delà de chaque extrémité du tube externe (13).

3. Tube selon la revendication 1 ou 2, caractérisé en ce que le tube interne (11) et les tubes externes (13) sont faits d'une matière choisie dans le groupe constitué par le PTFE, la fibre de polyester de synthèse, et en ce que le PTFE renforcé et le polymère non compatible non biodégradable est un agent d'étanchéité siliconé.

4. Tube selon la revendication 3, caractérisé en ce que le tube externe (13) est en fibre de polyester de synthèse et en ce que le tube interne (11) est en PTFE renforcé.

5. Tube selon l'une quelconque des revendications précédentes, caractérisé en ce que le tube externe a un diamètre interne d'environ 6 mm à environ 10 mm et une épaisseur de paroi d'environ 0,5 mm à 1 mm, et en ce que le tube interne a un diamètre interne d'environ 4 mm à environ 8 mm et une épaisseur de paroi d'environ 0,5 mm à environ 1 mm.

6. Tube selon la revendication 5, caractérisé en ce que le tube externe a un diamètre interne d'environ 8 mm et une épaisseur de paroi d'environ 1 mm et en ce que le tube interne a un diamètre interne d'environ 6 mm et une épaisseur de paroi d'environ 1 mm.

7. Tube selon l'une quelconque des revendications précédentes, caractérisé en ce que l'adhésif polymérique a été introduit dans l'espace compris entre les tubes externe et interne et a été réticulé in situ.

9

13    15

11

*FIG. 1*

13

11        15

*FIG. 2*

*FIG. 3*

| | A vs B | p < 0.001 | B vs C | p < 0.05 |
|---|---|---|---|---|
| | A vs C | p < 0.001 | B vs D | p < 0.05 |
| | A vs D | p < 0.001 | C vs D | ns |